Europäisches Patentamt

European Patent Office

Office européen des brevets

(11) Veröffentlichungsnummer: **0 493 372 A2**

# EUROPÄISCHE PATENTANMELDUNG

(21) Anmeldenummer: **92105193.4**

(22) Anmeldetag: **20.04.89**

(51) Int. Cl.5: **A61L 2/10**, A61L 9/20, A61L 2/20

Diese Anmeldung is am 26 - 03 - 1992 als Teilanmeldung zu der unter INID-Kode 60 erwähnten Anmeldung eingereicht worden.

(30) Priorität: **23.04.88 DE 3813793**

(43) Veröffentlichungstag der Anmeldung: **01.07.92 Patentblatt 92/27**

(60) Veröffentlichungsnummer der früheren Anmeldung nach Art. 76 EPÜ: **0 412 977**

(84) Benannte Vertragsstaaten:
**AT BE CH FR GB IT LI NL SE**

(71) Anmelder: **KLEINSCHMIDT, Dieter**
**Selzerbachweg 23**
**W-6367 Karben(DE)**

(72) Erfinder: **KLEINSCHMIDT, Dieter**
**Selzerbachweg 23**
**W-6367 Karben(DE)**

(74) Vertreter: **Jochem, Bernd, Dipl.-Wirtsch.-Ing.**
**Patentanwälte Beyer & Jochem Postfach 17**
**01 45 Staufenstrasse 36**
**W-6000 Frankfurt/Main 1(DE)**

(54) **Verfahren und Vorrichtung zum Desinfizieren von Gegenständen.**

(57) Die Vorrichtung zum Desinfizieren von Gegenständen wird entweder in Form einer mobilen Einheit in Räumen eingesetzt, aus denen Ozon nicht entweichen kann, sie kann aber auch alternativ fest darin installiert sein. In jedem Fall besteht sie aus einem oder mehreren UV-Strahlern (62), die so ausgelegt sind, daß außerhalb des Strahlers Ozon gebildet wird. Die zu desinfizierenden Gegenstände werden somit gleichzeitig intensiv mit UV-Strahlen und Ozon beaufschlagt. Gesundheitsschädliche Auswirkungen des giftigen Ozons werden dadurch vermieden, daß die UV-Strahler/Ozonerzeuger (62) mit einer Katalysatoreinrichtung (74, 76) kombiniert sind, durch die hindurch nach einer Ozonbehandlung das Gasvolumen mittels eines Gebläses (60) umgewälzt wird. Bei der mobilen Einheit sind mehrere UV-Strahler (62) und ein Rohr (74) mit Katalysator (76) parallel zwischen zwei Ständern (64) montiert, und das Gebläse (60) ist in dem Rohr (74) angeordnet.

Fig.6

Die Erfindung betrifft ein Verfahren zum Desinfizieren von Gegenständen in einem verschließbaren Raum oder dieses Raums selbst.

Der Erfindung liegt die Aufgabe zugrunde, ohne Anwendung von Hitze und ohne gesundheitsschädliche Auswirkungen auch Gegenstände komplizierter Gestalt, insbesondere mit Hohlräumen, in die von außen Keime eindringen können, die aber für äußere Strahlen unerreichbar sind, desinfizieren zu können.

Die Lösung dieser Aufgabe ist dadurch gekennzeichnet, daß die Gegenstände und/oder der Raum mit wenigstens einem Ozon bildenden UV-Strahler bestrahlt und zusätzlich durch das dabei gebildete Ozon desinfiziert werden, welches während des Desinfizierens im Raum umgewälzt wird, und daß anschließend das Ozon durch eine ein- und ausschaltbare Katalysatoreinrichtung abgebaut wird.

Zur Durchführung dieses Verfahrens wird eine Vorrichtung vorgeschlagen, welche wenigstens einen Ozon bildenden UV-Strahler, ein Gebläse zum Umwälzen des Ozons während des Desinfizierens und eine ein- und ausschaltbare Katalysatoreinrichtung zum Abbau des Ozons aufweist.

Die neue Vorrichtung ist gleichzeitig einfach und sehr wirksam, weil sie mit einem einzigen Gerät, nämlich dem Ozon bildenden UV-Strahler, zwei verschiedene bakterizide Wirkungen, nämlich die der UV-Strahlung und die des Ozons, optimal ausnutzt, da die Behandlung in einem Raum stattfindet, aus dem das Ozon nicht entweichen kann. Es konzentriert sich während der Behandlung und dringt auch in Ecken, Winkel und Hohlräume ein, wo Bakterien, Pilze oder sonstige Keime für die UV-Strahlung unerreichbar wären. Zugleich wird auch auf den äusseren, bestrahlten Flächen eine wirkungsvollere Keimreduzierung erreicht als mit UV-Bestrahlung allein, weil die bakterizide Wirkung des Ozons hinzutritt. Diese wirkt in dem Raum auch noch längere Zeit nach dem Abschalten des UV-Strahlers, so daß eine wirtschaftliche Betriebsweise der Vorrichtung möglich ist. Durch die Katalysatoreinrichtung wird schließlich das gesundheitsschädliche Ozon auf sehr einfache Weise schnell und vollständig beseitigt, bevor der Raum wieder geöffnet wird, so daß das Ozon außerhalb des Behandlungsraums überhaupt nicht in Erscheinung tritt. Die Beaufschlagung unregelmäßiger Oberflächen mit Ozon wird durch wenigstens ein Gebläse zum Umwälzen des Ozons ohne Einschaltung des Katalysators gefördert.

Für das erfindungsgemäße Verfahren kann eine bewegliche Katalysatoreinrichtung in Form eines Rohrs mit Katalysator und Gebläse verwendet werden, bei welcher das Rohr im wesentlichen ebenso lang ist wie eine oder mehrere UV-Strahlerröhren und zusammen mit diesen zwischen zwei Ständern mit Ein- und Austrittsöffnungen für mittels des Gebläses durch das Rohr gepumptes Gas befestigt ist. In bevorzugter Ausgestaltung der Erfindung ist in diesem Fall das Gebläse in dem Rohr angeordnet. Dadurch ergibt sich der Vorteil einer kürzeren Baulänge. Die mobile Einheit paßt in kleinere Brutschränke und bietet eine bessere Ausleuchtung mit UV-Strahlung, da die Ständer auf beiden Seiten sehr schmal sein können.

Schließlich sieht eine weitere Verbesserung vor, daß neben den UV-Strahlerröhren Schutzstäbe an den Ständern befestigt sind. Dadurch ergibt sich eine stabile Konstruktion, auch wenn die Ständer Kunststoffgehäuse sind.

Ausführungsbeispiele der Erfindung werden nachstehend anhand der Zeichnung näher erläutert. Es zeigen:

Fig. 1      einen Längsschnitt durch eine mobile Vorrichtung zum Desinfizieren, die zum Einsetzen in einen dicht abschließbaren Schrank bestimmt ist;

Fig. 2      einen Querschnitt durch die Vorrichtung nach Fig. 1;

Fig. 3      einen Längsschnitt durch eine Materialschleuse mit einer fest installierten Vorrichtung zum Desinfizieren;

Fig. 4      einen vereinfachten Querschnitt durch die Materialschleuse nach Fig. 3;

Fig. 5      eine Frontansicht der Materialschleuse nach Fig. 3 und 4;

Fig. 6 u. 7      Ansichten entsprechend Fig. 1 und 2 eines abgewandelten Ausführungsbeispiels;

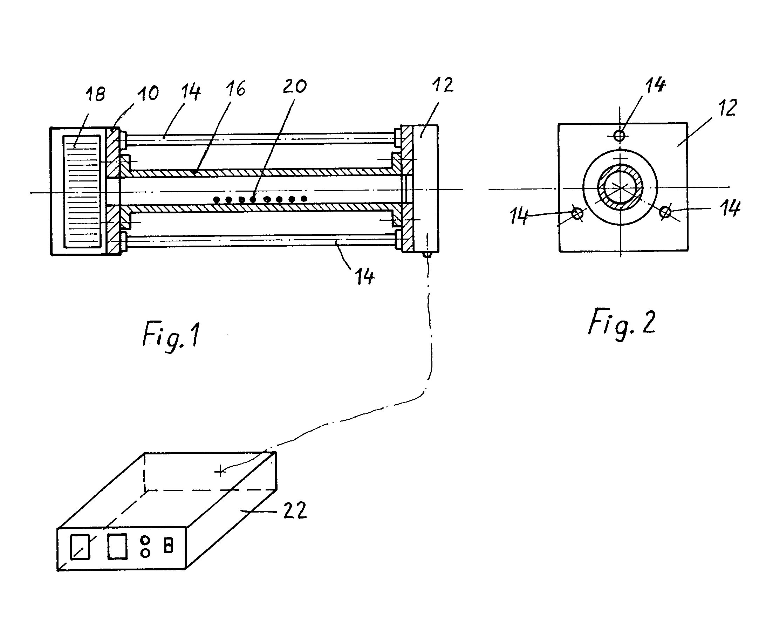

Die in Fig. 1 und 2 gezeigte mobile Vorrichtung zum Desinfizieren besteht im Beispielsfall aus einem linken quaderförmigen Ständer 10 und einem rechten, ebenfalls quaderförmigen Ständer 12. Bei paralleler Ausrichtung der Ständer 10, 12 erstrecken sich zwischen diesen drei röhrenförmige UV-Strahlerröhren 14. Ein über Endflanschen mit den Ständern 10 und 12 verschraubtes Rohr 16 sorgt einerseits für eine feste mechanische Verbindung der genannten Teile und bildet andererseits einen Strömungskanal, der von nicht gezeigten Lufteintrittsöffnungen im Ständer 12 zu ebenfalls nicht gezeigten Luftaustrittsöffnungen im Ständer 10 führt. Wenn ein im Ständer 10 gelagertes, durch einen Elektromotor antreibbares Gebläse 18 eingeschaltet wird, saugt dieses über die Lufteintrittsöffnung im Ständer 12 Luft aus dem umgebenden Raum an, zieht sie durch das Rohr 16 über einen dort z.B. in Form von Granulat vorliegenden Katalysator 20 und stößt sie über die Luftaustrittsöffnungen im Ständer 10 wieder in den umgebenden Raum aus. In der Luft enthaltenes Ozon wird dabei durch den Katalysator 20 in Luftsauerstoff $O_2$ umgewandelt.

Die zeitliche Steuerung der UV-Strahler 14, des Gebläses 18, eventuell gewünschter Anzeige- und Warnleuchten sowie Verriegelungen an Schranktüren erfolgt durch eine bei 22 angedeutete Ablaufsteue-

2

rung, die eine Voreinstellung der Parameter der desinfizierenden Behandlung zuläßt.

Die mobile Vorrichtung nach Fig. 1 und 2 kann vorübergehend z.B. in einen $CO_2$-Brutschrank, eine verschließbare laminarflow-Bank oder einen anderen dicht verschließbaren Schrank eingesetzt werden, in welchem Gegenstände und/oder die Innenwände desinfiziert werden sollen. Nach der Behandlung kann die Vorrichtung nach Fig. 1 und 2 wieder aus dem Schrank entnommen und in einen anderen Schrank eingesetzt werden.

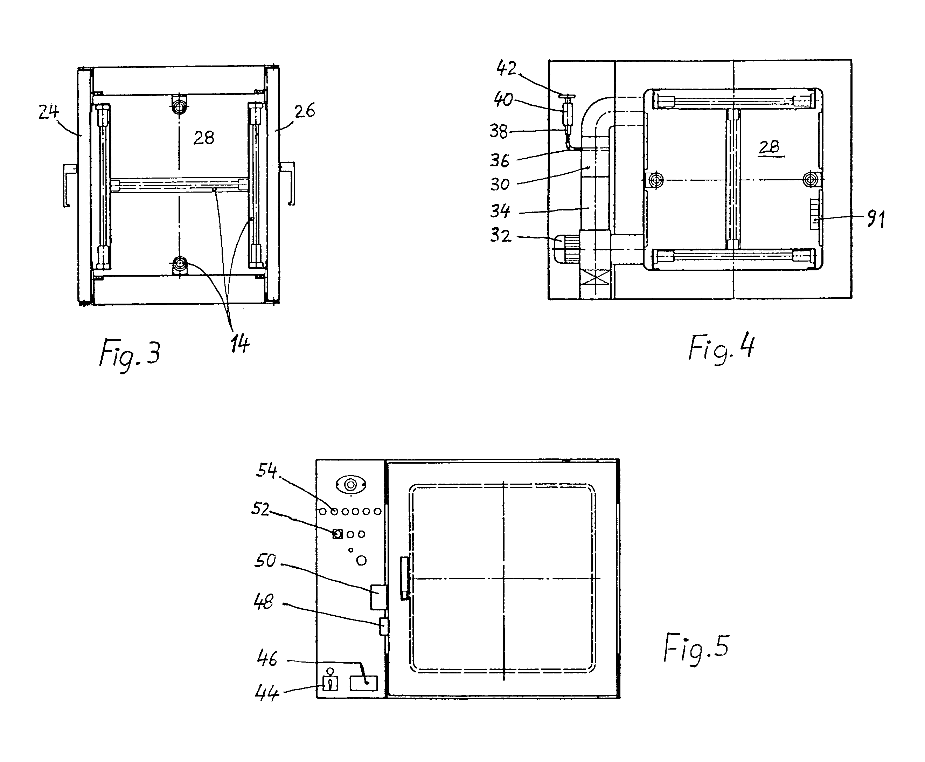

In Fig. 3 bis 5 ist dagegen ein Schrank - in diesem Fall eine Materialschleuse - mit einer fest installierten Vorrichtung zum Desinfizieren dargestellt. Die Materialschleuse ist z.B. in die Wand zwischen zwei Gebäuderäumen eingebaut und durch zwei gegenüberliegende Türen 24 und 26 von beiden Gebäuderäumen aus zugänglich. Wie aus Fig. 3 und 4 ersichtlich, sind sowohl an den Türen als auch an den Seitenwänden, dem Boden und der Decke UV-Strahlerröhren 14 angebracht. Der mit 28 bezeichnete Innenraum ist allseitig mit reflektierendem Metallblech ausgekleidet, um einen möglichst hohen Anteil der UV-Strahlung auf die zu desinfizierenden Gegenstände zu reflektieren. Diese ruhen während der Behandlung z.B. auf nicht gezeigten Drahtgitterböden, die z.B. auf Leisten an den Seitenwänden des Innenraums 28 liegen, leicht herausgenommen und auf einem anderen Niveau wieder eingesetzt werden können.

Die Katalysatoreinrichtung befindet sich in diesem Fall gemäß Fig. 4 neben dem Innenraum 28. Sie besteht aus einem Strömungskanal 30, der über Lufteintrittsöffnungen in einer Seitenwand des Innenraums 28 oben an diesen angeschlossen ist und unten durch Luftaustrittsöffnungen wieder darin mündet. Bei 32 ist eine Motor-Gebläseeinheit gezeigt, die im eingeschalteten Zustand für eine Umwälzung des Gasvolumens im Innenraum 28 durch den Strömungskanal 30 hindurch sorgt. Selbstverständlich kann die Strö-mungsrichtung auch umgekehrt gewählt werden. Wesentlich ist nur, daß nach einer gewissen Umwälzzeit das gesamte Gasvolumen des Innenraums 28 ein- oder mehrmals durch den Strömungskanal 30 hindurch umgewälzt und dabei mit einem darin bei 34 angedeuteten Katalysator in Berührung gekommen ist, durch welchen Ozon in Luftsauerstoff $O_2$ umgewandelt wird.

Um einen Druckausgleich zwischen dem Innenraum 28 und der Außenatmosphäre zu gewährleisten, ist eine nach außen führende Leitung 36 vorgesehen, in der ebenfalls ein Katalysator 38 und daneben außerdem ein Aktivkohlefilter 40 und ein Sterilfilter 42 angeordnet sind.

Die Materialschleuse hat gemäß Fig. 5 auf ihrer einen Frontseite einen Hauptschalter 44 mit Meldeleuchte und außerdem einen Betriebsstundenzähler 46. Darüber befinden sich auf beiden Türseiten Sicherheitsschalter 48 und jeweils eine elektrische Türverriegelung 50. Schließlich sind eine Starttaste 52 und zahlreiche Kontroll- und Meldeleuchten 54 vorhanden, die anzeigen, ob die UV-Strahler 14 oder die Katalysatoreinrichtung 32, 34 arbeiten.

Die vorstehend erwähnten UV-Strahler 14 sind vorzugsweise Niederdruck-Quecksilberdampfentladungslampen, die nach Größe und Leistung den unter der Handelsmarke Sterisol erhältlichen Heißkathodenstrahlern entsprechen, jedoch nicht mit dem bei diesen verwendeten Spezialglas hergestellt sind, welches die Ozonbildung verhindert. Im Gegenteil, die UV-Strahler 14 der erfindungsgemäßen Vorrichtung sind, unabhängig von ihrer Ausführung im einzelnen, gezielt so auszulegen, daß außerhalb der Strahler Ozon gebildet wird.

Die oben beschriebenen Katalysatoren 20 und 34 können jegliches Material enthalten, durch welches Ozon wirksam in Sauerstoff $O_2$ umgewandelt wird. In einer praktischen Ausführungsform enthält der Katalysator auf einem Aluminiumträger ungefähr 0,1 % Paladium und 0,2 % Platin.

Um die Wirksamkeit der neuen Vorrichtung zum Desinfizieren zu untersuchen, wurde die nachstehend schematisch dargestellte Behandlung einer Keimsuspension und parallel dazu eine Blindprobe vorgenom-men.

```
              5 ml   sterile Kochsalzlösung
                              |
                              ↓
              St. aureus,  Ps. aeruginosa
              Cand. albicans, E - coli
            ←                              →

2 ml Keimsuspension              2 ml Keimsuspension


UV/ O₃ - Behandlung              Blindprobe
        |                               |
        ↓                               ↓
6h - Begasung                    6h - Trockenschrank
Temp. ca. 50°C                   Temp. ca. 50°C
        |                               |
        ↓                               ↓
Aufschlämmen mit                 Aufschlämmen mit
5 ml steriler NaCl               5 ml steriler NaCl
        |                               |
        ↓                               ↓
1 ml Keimsuspension              1 ml Keimsuspension
überimpfen auf                   überimpfen auf
        |                               |
        ↓                               ↓
Blut - Agar                      Blut - Agar
CASO - Agar                      CASO - Agar
Sabourand - Agar                 Sabourand - Agar
        |                               |
        ↓                               ↓
Bebrütung 24 h                   Bebrütung 24 h
bei 37°C                         bei 37°C
        |                               |
        ↓                               ↓
Auswertung                       Auswertung
```

Bei der Auswertung stellte sich heraus, daß nach der Behandlung mit UV-Strahlen und Ozon überhaupt keine koloniebildenden Einheiten (kein Wachstum) festzustellen waren, während nach dem Blindversuch das Ergebnis Wachstum positiv (Platte dicht) lautete.

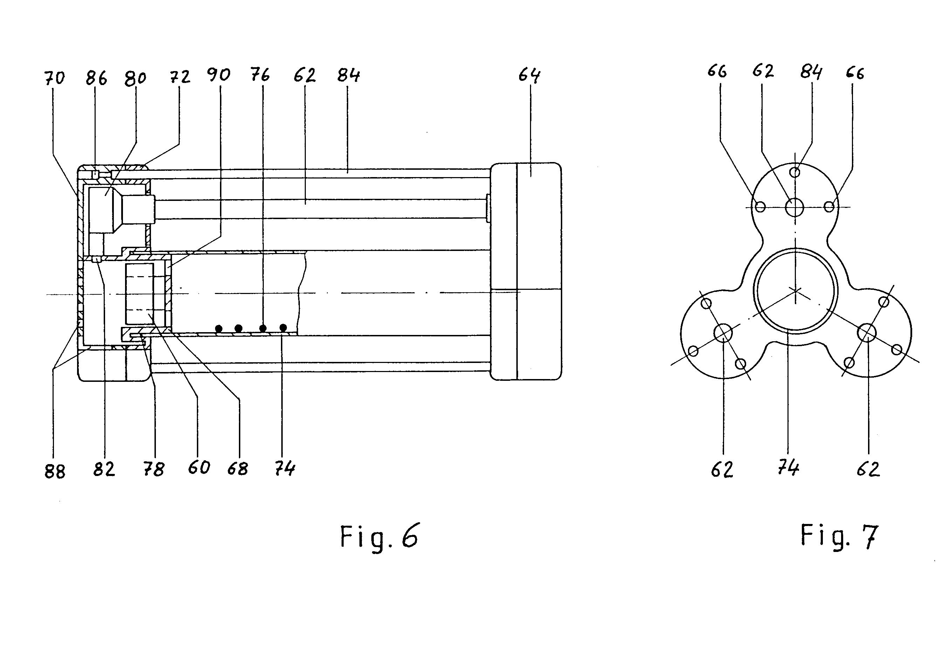

In Fig. 6 und 7 ist eine mobile Vorrichtung zum Desinfizieren ähnlich Fig. 1 und 2 gezeigt, die noch einfacher herzustellen ist. Ein weiterer Vorteil besteht darin, daß ein Gebläse 60 zwischen UV-Strahlerröhren 62 angeordnet ist. Somit kann die Länge der Vorrichtung im wesentlichen bis auf die Länge der nunmehr allein maßbestimmenden UV-Strahlerröhren 62 reduziert werden. Dieses ist besonders dann vorteilhaft, wenn z. B. die Brutschränke relativ klein sind und großflächig ausgeleuchtet werden sollen.

Die Vorrichtung nach Fig. 6 und 7 besteht aus linken und rechten Ständern 64, die durch Distanzstäbe 66 zusammengehalten werden. Auf der linken Seite ist der Ständer 64 im Schnitt gezeigt. Er besteht nur aus drei in der Form verschiedenen Kunststoffspritzteilen, nämlich einem Gehäuse 68, einem Deckel 70 und drei Ecken 72. Die Distanzstäbe 66, im Beispielsfall sechs Stück, sind mit dem Gehäuse 68 fest verschraubt. Das Rohr 74 mit dem eingeschlossenen Katalysator 76 ist in einem Ringschlitz 78 des Gehäuses 68 geführt und gehalten. Weiterhin sind am Gehäuse 68 Fassungen 80 an Durchbrüchen 82 befestigt. Sie halten die UV-Strahlerröhren 62 in Position. Somit übernimmt das Gehäuse 68 mit den Distanzstäben 66 eine Trägerfunktion für die gesamte Vorrichtung. Der Deckel 70 und die Ecken 72 übernehmen nur Abdeckfunktionen, wobei der rechte und linke Deckel 70 mit drei Stäben 84 verbunden und mittels Schrauben 86 verschraubt ist. Hierdurch werden gleichzeitig die Ecken 72 gehalten.

Um defekte UV-Strahler auszutauschen, bedarf es nur des Lösens der Schrauben 86. Hierdurch können Deckel 70 und Ecken 72 abgenommen werden und ermöglichen einen leichten Zugriff zu der Fassung 80.

Die Stäbe 66 und 84 sind nicht nur als Befestigungsstäbe gedacht, sondern haben gleichzeitig durch die sternartige Anordnung um die UV-Strahler eine schützende Aufgabe.

Wenn das Gebläse 60 eingeschaltet wird, saugt es über Lufteintrittsöffnungen 88 Luft aus dem umgebenden Raum an und drückt sie dann durch Schlitze 90, das Rohr 74 und über den Katalysator 76. Diese Luft tritt auf der anderen Seite wieder in den umgebenden Raum aus. Die seitlich und stirnseitig angeordneten Lufteintrittsöffnungen 88 ermöglichen es, daß die Vorrichtung lageunabhängig ist und somit auch hochkant gestellt werden kann. Ein weiterer Vorteil ist, daß die die Ständer 64 bildenden Kuststoffteile 68, 70, 72 unabhängig von den jeweils verwendeten Strahlern 62 sind, denn bei anderen Längen der UV-Strahler 62 werden nur ein in der Länge darauf abgestimmtes Rohr 74 und abgestimmte Stäbe 66, 84 benötigt.

An einer geeigneten Stelle im Behandlungsraum ist, wie z. B. in Fig. 4 gezeigt, ein Gebläse 91 angeordnet, welches für Umwälzung des Ozons sorgt, ohne daß dieses dabei durch den Katalysator strömt.

## Patentansprüche

1. Verfahren zum Desinfizieren von Gegenständen in einem verschließbaren Raum oder dieses Raums selbst, **dadurch gekennzeichnet**, daß die Gegenstände und/oder der Raum mit wenigstens einem Ozon bildenden UV-Strahler (14, 62) bestrahlt und zusätzlich durch das dabei gebildete Ozon desinfiziert werden, welches während des Desinfizierens im Raum umgewälzt wird, und daß anschließend das Ozon durch eine ein- und ausschaltbare Katalysatoreinrichtung (16, 18, 20; 30, 32, 34; 60, 74, 76) abgebaut wird.

2. Vorrichtung zum Desinfizieren von Gegenständen in einem verschließbaren Raum oder dieses Raums selbst, **dadurch gekennzeichnet**, daß sie wenigstens einen Ozon bildenden UV-Strahler (14, 62), ein Gebläse (91) zum Umwälzen des Ozons während des Desinfizierens und eine ein- und ausschaltbare Katalysatoreinrichtung (16, 18, 20; 30, 32, 34; 60, 74, 76) zum Abbau des Ozons aufweist.

3. Vorrichtung zum Desinfizieren von Gegenständen in einem verschließbaren Raum oder dieses Raums selbst, **dadurch gekennzeichnet**, daß sie wenigstens einen Ozon bildenden UV-Strahler (14, 62) und eine ein- und ausschaltbare Katalysatoreinrichtung (16, 18, 20; 30, 32, 34; 60, 74, 76) zum Abbau des Ozons aufweist, welche die Form eines Rohrs (16, 74) mit Katalysator (20, 76) und Gebläse (18, 60) hat, wobei das Rohr (16, 74) im wesentlichen ebenso lang ist wie eine oder mehrere UV-Strahlerröhren (14, 62) und zusammen mit diesen zwischen zwei Ständern (12, 64) mit Ein- und Austrittsöffnungen für mittels des Gebläses (18, 60) durch das Rohr (16, 74) gepumptes Gas befestigt ist, und wobei das Gebläse (60) in dem Rohr (74) angeordnet ist.

4. Vorrichtung zum Desinfizieren von Gegenständen in einem verschließbaren Raum oder dieses Raums selbst, **dadurch gekennzeichnet**, daß sie wenigstens einen Ozon bildenden UV-Strahler (14, 62) und eine ein- und ausschaltbare Katalysatoreinrichtung (16, 18, 20; 30, 32, 34; 60, 74, 76) zum Abbau des Ozons aufweist, welche die Form eines Rohrs (16, 74) mit Katalysator (20, 76) und Gebläse (18, 60) hat, wobei das Rohr (16, 74) im wesentlichen ebenso lang ist wie eine oder mehrere UV-Strahlerröhren (62) und zusammen mit diesen zwischen zwei Ständern (12, 64) mit Ein- und Austrittsöffnungen für mittels des Gebläses (18, 60) durch das Rohr (16, 74) gepumptes Gas befestigt ist, wobei neben den UV-Strahlerröhren (62) Schutzstäbe (66, 84) an den Ständern (64) befestigt sind.

Fig. 2

Fig. 1

Fig. 4

28

91

42
40
38
36
30
34
32

Fig. 5

54
52
50
48
46
44

Fig. 3

26

28

14

24

Fig. 7

Fig. 6